# EUROPÄISCHE PATENTANMELDUNG

(11) **EP 4 454 635 A1**
(43) Veröffentlichungstag der Anmeldung: **30.10.2024**
(21) Anmeldenummer: 24173061.3
(22) Anmeldetag: 29.04.2024
(51) Int. Cl.: A61K 8/06, A61K 8/20, A61Q 19/00, A61Q 19/10

(54) **KOSMETISCHE ZUBEREITUNG MIT VERBESSERTER KONSERVIERUNG**

(30) Priorität: 29.04.2023 DE 102023111166
(71) Anmelder: Inavive Lab Z.A. du Val d'Huisne INAVIVE Lab, 28400 Nogent Le Rotrou (FR)
(72) Erfinder: Guérin, Didier, 31540 Saint Felix Lauragais (FR); Ott, Peter, 66839 Schmelz (DE); Di Salvo, Morgane, 13012 Marseille (FR)
(74) Vertreter: Bosch Jehle Patentanwaltsgesellschaft mbH

(57) **Zusammenfassung**

Die Erfindung betrifft eine kosmetische Zubereitung enthaltend eine wässrige Phase. Dabei umfasst die wässrige Phase Wasser und ein aktiviertes Ultrareinwasser, wobei der wässrigen Phase das Wasser und das aktivierte Ultrareinwasser zugegeben werden, und wobei das aktivierte Ultrareinwasser vor der Zugabe zur wässrigen Phase einen Gehalt von 60 bis 1500 mg/l ClO⁻ -Ionen und ein Redoxpotential von + 700 bis + 950 mV aufweist. Der Anteil an aktiviertem Ultrareinwasser beträgt mindestens 70 bis 99,9% des zu der wässrigen Phase insgesamt zugegebenen Wassers und aktivierten Ultrareinwassers.

Weiterhin betrifft die Erfindung die Verwendung von aktiviertem Ultrareinwasser, das einen Gehalt von 60 bis 1500 mg/l ClO⁻ -Ionen und ein Redoxpotential von + 700 bis + 950 mV aufweist, zur Herstellung einer erfindungsgemäßen kosmetischen Zubereitung.

## Beschreibung

Die vorliegende Erfindung betrifft eine kosmetische Zusammensetzung enthaltend eine wässrige Phase.

Kosmetische Zubereitungen sind weit verbreitet und erfreuen sich großer Beliebtheit. Es sind Stoffe oder Stoffgemische, die äußerlich mit den Teilen des menschlichen Körpers (Haut Behaarungssystem, Nägel, Lippen und äußere intime Regionen) oder mit den Zähnen und den Schleimhäuten der Mundhöhle in Berührung kommen und ausschließlich oder überwiegend der Reinigung, der Pflege oder dem Schutz der Haut und der Haare dienen. Ferner werden sie zur Desodorierung und Parfümierung des Körpers und zur Veränderung des Aussehens eingesetzt. Insofern haben viele kosmetische Zubereitungen verbessernden bzw. verfeinernden Einfluss auf das Hautbild und helfen bei der Erhaltung eines guten Hautzustandes.

Hierzu enthalten die kosmetischen Zubereitungen als Hauptbestandteile Wasser und/oder verschiedenes Öl. Diese beiden sogenannten Phasen, die Wasserphase und die Ölphase, fungieren als Produktgrundlage der kosmetischen Zubereitung und weisen als Träger - und Basiskomponenten je nach Bedarf verschiedene, pflegende, duftende oder färbende Zusatz- und Inhaltsstoffe auf.

Es sind nun Produkte bekannt, die als rein ölhaltige Zubereitungen keine Wasserphase aufweisen, wie beispielsweise Salben oder Lipo-Gele.

Insbesondere für die Hautpflege werden aber überwiegend solche Zubereitungen verwendet, die entweder alleine eine wässrige Phase aufweisen oder deren Grundlage aus wässriger und öliger Phase bestehen. Erstere wären z.B. Lösungen, Haut- oder Haarwässer, Hydrogele.

Wenn nun beide Basiskomponenten, Wasser und Öl, in der kosmetischen Zubereitung enthalten sind, können sie in sehr unterschiedlichen Anteilen vorhanden sein. Unabhängig davon sind diese beiden Komponenten von Natur aus nicht miteinander mischbar, weshalb grenzflächenaktive Substanzen, die sogenannten Emulgatoren, zugesetzt werden, um stabile Zubereitungen zu erhalten, bei denen sich Wasser- und Ölphase nicht voneinander trennen. In Abhängigkeit vom Mengenverhältnis von Wasser- und Ölphase, aber insbesondere auch in Abhängigkeit von den Eigenschaften des verwendeten Emulgators oder Emulgatorgemisches können Wasser-in-Öl-Emulsionen (W/O) oder Öl-in-Wasser-Emulsionen (O/W) entstehen.

Bei der W/O-Emulsion bildet die Ölphase die äußere Phase, in der die wässrige Komponente als fein verteilte Tröpfchen vorliegt und umhüllt wird. Da das Öl als äußere Phase vorliegt, hat die Zubereitung das Erscheinungsbild einer reichhaltigen Creme oder Fettcreme. Derartige Zubereitungen eignen sich eher für die Pflege oder den Schutz von trockener Haut.

Als O/W-Emulsionen werden hingegen solche Zubereitungen bezeichnet, deren äußere Phase die wässrige Phase ist, in der feine Öltröpfchen verteilt sind. Dieser Emulsionstyp zeichnet sich, insbesondere aufgrund der wässrigen äußeren Phase, durch anwenderfreundliche Eigenschaften aus. So sind eine Creme, Lotion, Körpermilch oder andere kosmetische Zubereitungen mit wässriger Außenphase und öliger Innenphase leicht auf der Haut zu verteilen. Sie ziehen schnell in die Haut ein und haben eine angenehme Haptik. Da sie in der Regel keinen Fettfilm hinterlassen, eignen sich diese Zubereitungen für ein weites Anwendungsfeld, insbesondere für normale oder fettige Haut.

Weiterhin stehen auch Mehrfachemulsionen zur Verfügung, bei denen die unterschiedlichen Phasen in (mehrfach) ineinander gelagerten konzentrischen Tröpfchen vorliegen oder auch solche Emulsionen, die eine innere feste Phase enthalten können. Auch hier entscheidet die Qualität der äußeren Phase, wässrig oder ölig, über die resultierenden Anwendungseigenschaften

Die wässrige und die ölige Komponente der Grundlage enthalten unterschiedliche Inhaltsstoffe, die den jeweiligen kosmetischen Zubereitungen deren reinigende, pflegende, (haut- oder haar-)schützende, desodorierende oder farbgebende Eigenschaften verleihen oder diese unterstützen.

Die kosmetischen Zubereitungen mit wässriger Phase sind anfällig für mikrobielle Verunreinigung, Befall und Verderb. Insbesondere betroffen sind die O/W-Emulsionen mit Wasser in der Außenphase, da bei ihnen das Wasser in einer für Mikroorganismen von außen leicht zugänglichen Form enthalten ist. Aber auch wenn das Wasser in der Innenphase vorliegt, unterliegt es der Gefahr der Verkeimung.

Daher müssen diese kosmetischen Zubereitungen zum Schutz vor mikrobieller Kontamination, Keimwachstum und Verderben entsprechend konserviert werden.

Hierzu werden üblicherweise unterschiedliche Konservierungsstoffe eingesetzt. Sie verhindern bestimmte chemische Reaktionen, wie Oxidation, oder mikrobiologische Veränderungen und gewährleisten somit, dass eine Zubereitung in einer ungeöffneten Verpackung über eine ausgewählte Lagerzeit nicht verdirbt. Zusätzlich bieten Konservierungsstoffe einen gewissen Schutz von Verkeimung der in Gebrauch befindlichen Zubereitung in der geöffneten Verpackung durch den Eintrag von Mikroorganismen bei der bestimmungsgemäßen Entnahme.

Es werden hierbei verschiedene Konservierungsstoffe verwendet. Sie gehören teilweise zu den hochallergenen Stoffen, teilweise besitzen sie potentiell allergieauslösende Eigenschaften.

Darüber hinaus fallen manche der üblicherweise verwendeten Konservierungsstoffe in die Gruppe der endokrinen Disruptoren. Dies sind endokrin wirksame Substanzen, die auch als "Umwelthormone" bezeichnet werden. Im Körper können sie auch in geringen Mengen durch Veränderung des Hormonsystems die Gesundheit beeinträchtigen oder schädigen.

Seit einiger Zeit erhöht sich die Anzahl derjenigen Anwender, die auf einen oder mehrere dieser Konservierungsstoffe empfindlich reagieren und Hautirritationen oder allergische Reaktionen entwickeln.

Außerdem ist bei den Verbrauchern eine zunehmende Tendenz zu ökologisch bewusstem Verhalten zu beobachten, das die Verwendung von möglichst naturbelassenen kosmetischen Zubereitungen und die weitestgehende Vermeidung von Konservierungsstoffen in kosmetischen Zubereitungen beinhaltet.

Die Reduktion der Konzentration von Konservierungsstoffen durch Kombination mehrerer Stoffe in einer kosmetischen Zubereitung ist grundsätzlich bekannt. So sind im Stand der Technik viele verschiedene Kombinationen von Konservierungsstoffen und deren möglicher Einsatz in niedrigeren Konzentrationen als bei Verwendung der Einzelsubstanzen beschrieben.

Aus der JP 2019-210250 A ist eine Zusammensetzung eines konservierenden Hilfsstoffs mit einem konservierenden Wirkstoff bekannt, wobei der Hilfsstoff (i) Isopentyldiol oder (ii) Isopentyldiol und 1,2-Hexandiol ist, und der Wirkstoff mindestens einen Stoff ausgewählt aus der Gruppe enthaltend 2-Phenoxyehanol, Caprylylglycol und Ethylhexylglycerin ist.

In der WO 2013/091978 (A2) wird eine antimikrobielle Zusammensetzung und ein diese Zusammensetzung enthaltendes kosmetisches Produkt beschrieben. Die Zusammensetzung enthält Ethyllauroylarginat und mindestens einen Monoglycerylether der Formel 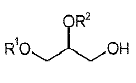 wobei einer der beiden Reste R1 und R2 eine verzweigte oder lineare C3- bis C12-Alkylgruppe und der andere ein H-Atom darstellt.

Weiterhin werden Konservierungsstoffe pflanzlichen Ursprungs im Stand der Technik genannt, so z.B. in der CN 10 4738033 (A), die eine Zusammensetzung von pflanzlichen Konservierungsstoffen, die Citral, Vanillin, Anissäure und Magnolol umfasst, offenbart.

Auch aus der KR 2008/0011546 (A) ist eine kosmetische Zusammensetzung bekannt, die in der Lage ist Irritationen durch chemische Konservierungsstoffe zu entfernen, indem ein Extrakt von Lonicera-Blüten anstatt eines chemischen Konservierungsstoff enthalten ist.

Nun ist es so, dass einerseits auch pflanzliche Inhaltsstoffe Allergien auslösen können und dass andererseits die Gesamtkonzentration an Konservierungsstoff bei der Kombination mehrerer Substanzen, die je einzeln in kleiner Konzentration enthalten sind, in der Summe doch wieder ein größeres Ausmaß annimmt.

Daher ist es wünschenswert, sowohl die Anzahl als auch die Konzentration der in kosmetischen Zubereitungen verwendeten Konservierungsstoffe möglichst gering zu halten. Gleichzeitig muss aber eine mikrobielle Kontamination, eine Verkeimung und das Keimwachstum in kosmetischen Zubereitungen zuverlässig verhindert werden, um einen Verderb der Zubereitung und Schäden durch die Anwendung zu vermeiden.

Es ist daher die Aufgabe der vorliegenden Erfindung, eine kosmetische Zubereitung zur Verfügung zu stellen, die einen verminderten Gehalt an Konservierungsmitteln aufweist und trotzdem gegen mikrobielle Verkeimung unempfindlich ist.

Die vorliegende Erfindung löst diese Aufgabe einer kosmetischen Zubereitung, die eine wässrige Phase enthält, dadurch, dass die wässrige Phase Wasser und ein aktiviertes Ultrareinwasser umfasst, wobei der wässrigen Phase das Wasser und das aktivierte Ultrareinwasser zugegeben werden, und wobei das aktivierte Ultrareinwasser vor der Zugabe zur wässrigen Phase einen Gehalt von 60 bis 1500 mg/l ClO⁻-Ionen und ein Redoxpotential von + 700 bis + 950 mV aufweist, wobei der Anteil an aktiviertem Ultrareinwasser mindestens 70 bis 99,9% des zu der wässrigen Phase insgesamt zugegebenen Wassers und aktivierten Ultrareinwassers beträgt.

Das Wasser der wässrigen Phase kann ein im Verlauf der Herstellung zugesetztes Wasser sein. Hierfür sind beispielsweise Wasser für kosmetische Zwecke, Aqua purificata oder destilliertes Wasser geeignet. Außerdem kann dieses Wasser auch in Form von anderen möglichen Inhaltsstoffen, die als wasserhaltige Flüssigkeiten vorliegen und zugegeben werden, zu der kosmetischen Zubereitung zugegeben werden. Dies beispielsweise, wenn wässrige Pflanzenextrakte oder Filmbildner, Gelbildner, wie Cellulose- oder Stärkezusammensetzungen, oder Stabilisatoren, Puffer oder pH-Regulatoren, die in wässriger Form als Rohstoffe vorliegen, Teil einer Rezeptur sind.

Das aktivierte Ultrareinwasser ist ein Wasser, das einen Gehalt von 60 bis 1500 mg/l ClO⁻ -Ionen und ein Oxidationspotential von + 700 bis + 950 mV aufweist und in einem mehrstufigen elektrochemischen Verfahren hergestellt wird. Es ist beispielsweise erhältlich bei POC Consulting GmbH, Schmelz. In Hinsicht auf das aktivierte Ultrareinwasser und das Verfahren zu dessen Herstellung wird Bezug genommen auf die Anmeldung EP 4019476, deren Inhalt hiermit in diese Anmeldung aufgenommen wird.

Das aktivierte Ultrareinwasser im Sinn der hier vorliegenden Anmeldung hat einen Gehalt von 60 bis 1500 mg/l ClO⁻ -Ionen. Insbesondere hat es einen Gehalt von beispielsweise 60 mg/l, 600 mg/l, 800 mg/l, 1200 mg/l oder 1500 mg/l ClO⁻ -Ionen. Bevorzugt ist ein Gehalt von 800mg/l oder 1200 mg/. Für eine kosmetische Zubereitung mit einem hohen Anteil an aktiviertem Ultrareinwasser, wie 90 bis 99,9 % ist ein Gehalt von 60 mg/l bevorzugt.

Es ist dabei erhältlich, indem eine mit herkömmlichem Ultrareinwasser (das sich insbesondere durch einen niedrigen TOC-Grenzwert und eine niedrige Leitfähigkeit auszeichnet) verdünnte Lösung von hochreinem Chloridsalz, wie beispielsweise NaCl, durch Elektrolyse in einer zyklischen Elektrolysezelle behandelt wird. Die Elektrolyse kann dabei hinsichtlich verschiedener Verfahrensparameter, wie Spannung oder Stromfluss, sowie Anordnung und Geometrie der Elektroden und auch der Verweildauer der Lösung in der zyklischen Elektrolysezelle gesteuert werden und somit können die oben genannten unterschiedlichen Gehalte an ClO⁻ - Ionen eingestellt werden. Insbesondere wird als zyklische Elektrolysezelle eine Zelle verwendet, die keinen durch Membran oder Diaphragma getrennten Kathoden- und Anodenraum aufweist.

Dabei ist die Lösung des hochreinen Chloridsalzes mit einem ultrareinen elektrochemisch modifizierten Wasser als Ausgangsstoff hergestellt. Dieses ultrareine elektrochemisch modifizierte Wasser wird erhalten, indem herkömmliches Ultrareinwasser in mehreren Verfahrensschritten (1) entgast, (2) in einer Durchfluss-Elektrolysezelle, die keinen getrennten Kathoden- und Anodenraum aufweist, also ohne Membran oder Diaphragma, mit einer DC-Spannung beaufschlagt, (3) mit Umgebungsluft und/oder O₂ versetzt wird und das Wasser mit der Umgebungsluft und/oder dem O₂ an einer Oberfläche zur Reaktion gebracht wird und abschließend nochmals (4) entgast wird. Die Verfahrensschritte (2) und (3) können dabei einfach oder mehrfach wiederholt werden.

Die Verwendung des ultrareinen elektrochemisch modifizierten Wassers zur Herstellung einer Chloridsalzlösung und die Elektrolyse der so erhaltenen, mit herkömmlichem Ultrareinwasser verdünnten Chloridsalzlösung führen dazu, dass ein aktiviertes Ultrareinwasser erhalten wird, das den genannten Gehalt an ClO⁻ - Ionen und das genannte Redoxpotential aufweist. Insbesondere ein Gehalt von 60 mg/l ClO⁻ -Ionen ist bevorzugt. Ein Großteil der ClO⁻ -Ionen reagiert bald nach Zugabe des aktivierten Ultrareinwassers zur wässrigen Phase der kosmetischen Zubereitung zu NaCl. Die kosmetische Zubereitung ist somit nahezu frei von ClO⁻ - Ionen.

Weiterhin kann die kosmetische Zubereitung in der wässrigen Phase einen Konservierungsstoff umfassen. Sie kann auch in der öligen Phase einen Konservierungsstoff enthalten, vor allem die wässrige Phase ist aber anfällig für eine mikrobielle Kontamination. Hierbei können alle Konservierungsstoffe zum Einsatz kommen, die in einer kosmetischen Zubereitung geeignet sind, ausschließlich oder überwiegend die Entwicklung von Mikroorganismen zu hemmen. Nicht unter diesen Begriff fallen im Rahmen der vorliegenden Erfindung solche Stoffe, die zwar das Keimwachstum hemmen, die aber überwiegend zu einem anderen Zweck, wie beispielsweise als Wirkstoff für die Hautpflege, als Mittel zum Schutz vor reaktiven Sauerstoffspezies, als Stabilisator oder als Puffer der kosmetischen Zubereitung zugesetzt sind.

Gängige Konservierungsstoffe sind beispielsweise in der Verordnung (EG) Nr. 1223/2009 in Anhang V aufgelistet.

Für die vorliegende Erfindung bevorzugte Konservierungsstoffe sind beispielsweise Benzoesäure und deren Natrium-, Calcium-, Kalium-, Magnesium- oder Ammoniumsalz; ferner Benzoesäureester, wie Ethyl-, Methyl-, Propyl-, Butyl-, Isopropyl-, Isobutyl- oder Phenylbenzoat oder Benzoesäure-Monoethanolaminsalz. Für die erfindungsgemäße kosmetische Zubereitung sind außerdem aufgrund ihres geringen Allergiepotentials Sorbinsäure und deren Calcium-, Natrium- oder Kaliumsalz geeignet, sowie 4-Hydroxybenzoesäure und deren Ester. Auch Dehydroacetsäure und Natriumdehydroacetat, o-Cymen-5-ol, 2-Phenoxyethanol, Benzylalkohol, Chlorphenesin, Chlorhexidin und dessen Diacetat, Digluconat oder Dihydrochlorid werden bevorzugt als Konservierungsstoffe im Sinne dieser Erfindung eingesetzt.

Als besonders bevorzugte Konservierungsstoffe werden wegen ihrer guten Verträglichkeit insbesondere Benzoesäure und Benzoesäureester, Sorbinsäure und deren Salze, Chlorphenesin und Phenoxyethanol verwendet.

Für die Zugabe eines Konservierungsstoffs oder eines Gemischs von verschiedenen Konservierungsstoffen existieren zulässige Höchstkonzentrationen, die bei deren Zugabe nicht überschritten werden dürfen. Die Höchstkonzentration kann dabei insbesondere als Masseanteil in Gew.-% angegeben werden.

Die für Konservierungsstoffe zulässigen Höchstkonzentrationen können unter anderem durch länderspezifische gesetzliche Vorschriften vorgegeben sein. Hierbei sind in unterschiedlichen Ländern unterschiedliche Vorgaben denkbar. So wird von der FDA durch Erlass entsprechender Richtlinien der Gebrauch von Konservierungsstoffen für die USA geregelt. Eine mögliche Verordnung, die zulässige Höchstkonzentrationen für die EU verbindlich vorschreibt, ist beispielsweise die Verordnung (EG) Nr. 1223/2009 des Europäischen Parlaments und des Rates in ihrer jeweils aktuellen Fassung. Dort sind die in der EU in kosmetischen Zubereitungen zugelassenen Konservierungsstoffe im Anhang V abschließend aufgelistet. Hierbei ist zu den angeführten Konservierungsstoffen, deren Bezeichnung sich den Spalten "b" und "c" entnehmen lässt, für jeden Konservierungsstoff in Spalte "g" des Anhang V die jeweils zulässige Höchstkonzentration festgelegt, die sich auf eine aus Spalte "f" entnehmbare Art des Mittels bzw. spezifische Verwendung des Mittels bezieht.

Daher können die nach Verordnung (EG) Nr. 1223/2009 zulässigen Höchstkonzentrationen für einen Konservierungsstoff in Abhängigkeit der Verwendung der ihn enthaltenden Zubereitung unterschiedliche Werte aufweisen.

So ist beispielsweise - wie aus Tabelle 1 (Tab.1) ersichtlich - Benzoesäure in einer Höchstkonzentration von 2,5 % in auszuspülenden/abzuspülenden Mitteln (wie Haarshampoo oder Duschgel) zugelassen, in einer Hautcreme aber nur zu 0,5 %.

**Tab. 1: Auszug aus Verordnung (EG) Nr. 1223/2009, S. 349 Anhang V in kosmetischen Zubereitungen zugelassene Konservierungsstoffe**

| Laufende Nummer | Bezeichnung der Stoffe | | | | Bedingungen | |
|---|---|---|---|---|---|---|
| | Chemische Bezeichnung/ INN | Gemeinsame Bezeichnung im Glossar der Bestandteile | CAS-Nummer | EG-Nummer | Art des Mittels, Körperteile | Höchstkonzentration in der gebrauchsfertigen Zubereitung |
| a | b | c | d | e | f | g |
| 1 | Benzoesäure und ihr Natriumsalz | Benzoic acid | 65-85-0 | 200-618-2 | Auszuspülende/abzuspülende Mittel, ausgenommen Mundmittel | 2,5 % (Säure) |
| | | Sodium Benzoate | 532-32-1 | 208-534-8 | | |
| | | | | | Mundmittel | 1,7 % (Säure) |
| | | | | | Auf der Haut/im Haar verbleibende Mittel | 0,5 % (Säure) |
| 1a | Andere als die unter Nr. 1 genannten Salze der Benzoesäure und Benzoesäureester | Ammonium benzoate, calcium benzoate, potassium benzoate, magnesium benzoate, MEA-benzoate, methyl benzoate, ethyl benzoate, propyl benzoate, butyl benzoate, isobutyl benzoate, isopropyl benzoate, phenyl benzoate | 1863-63-4, 2090-05-3, 582-25-2, 553-70-9, 4337-66-0, 93-58-3, 93-89-0, 2315-68-6, 136-60-7, 120-50-3, 939-48-0, 93-99-2 | 217-468-9, 218-235-4, 209-481-3, 209-045-2, 224-387-2, 202-259-7, 202-284-3, 219-020-8, 205-252-7, 204.401.3, 213-361-6, 202-293-2 | | 0,5 % (Säure) |

Aufgrund des erfindungsgemäßen Einsatzes des aktivierten Ultrareinwassers ist es nun in einer bevorzugten Ausführungsform möglich, dass die Konzentration eines Konservierungsstoffs nur höchstens 35 % der für ihn zulässigen Höchstkonzentration beträgt. Dabei kann die genannte Höchstkonzentration auch abhängig sein von der Art der kosmetischen Zubereitung bzw. von der bestimmungsgemäßen Anwendung derselben.

Auch beim Einsatz von Konservierungsstoffgemischen weisen die Konzentrationen jedes Stoffes im Gemisch nur bis höchstens 35 % seiner zulässigen Höchstkonzentration auf.

Besonders bevorzugt ist es, wenn die Konzentration des Konservierungsstoffs in der kosmetischen Zubereitung lediglich bis zu 18 % der zulässigen Höchstkonzentration beträgt.

Weiterhin ist es denkbar, dass die kosmetische Zubereitung in einer Ausführungsform eine Konzentration des Konservierungsstoffs aufweist, die 7,5 bis 34 % der zulässigen Höchstkonzentration beträgt.

Ebenso kann die kosmetische Zubereitung so ausgestaltet sein, dass die Konzentration des Konservierungsstoffs 7,5 bis 18 % der zulässigen Höchstkonzentration beträgt.

Das zur wässrigen Phase der kosmetischen Zubereitung zugegebene aktivierte Ultrareinwasser kann vor der Zugabe bevorzugt einen pH-Wert von 8,2 - 9,5 aufweisen. Dieser kann durch das oben genannte Verfahren zur Herstellung des aktivierten Ultrareinwassers eingestellt werden. Ein derartiger pH-Wert ist insbesondere geeignet, um den angegebenen Gehalt an ClO⁻ -Ionen zu gewährleisten. Nach Zugabe des aktivierten Ultrareinwassers zur kosmetischen Zubereitung befindet sich der pH-Wert üblicherweise bei ca. pH 5 bis 6,5 oder er wird auf Werte zwischen pH 5,0 bis 5,5 mit pH-Regulatoren eingestellt.

Die erfindungsgemäße kosmetische Zubereitung kann rein wasserbasiert sein, d.h. sie weist dann nur eine wässrige Phase auf. Es ist aber gemäß einer bevorzugten Ausgestaltung auch denkbar, dass sie weiterhin eine ölige Phase umfasst. Hierbei ist es wiederum möglich, dass die Zubereitung als Wasser-in-Öl-Emulsion vorliegt. Es ist aber für diese Ausführungsform bevorzugt, dass die ölige Phase mit der wässrigen Phase eine Öl-in-Wasser-Emulsion bildet.

Die wässrige Phase der kosmetischen Zubereitung umfasst beispielsweise zusätzlich ein Lösungsmittel. Hierbei sind ein oder mehrere Lösungsmittel ausgewählt aus der Gruppe enthaltend Ethanol, Isopropanol, Glycerol, Propylenglycol, Butylenglycol Pentylenglycol und Hexylenglycol denkbar. Besonders bevorzugt sind 1,2-Propandiol und 1,2-Pentandiol. Diese Lösungsmittel wirken hautpflegend und binden Feuchtigkeit in der Zubereitung. Weiterhin kann mit Zugabe dieser Lösungsmittel die Viskosität der Zubereitung in einen gewünschten Bereich gebracht werden.

Die kosmetische Zubereitung umfasst bevorzugt einen weiteren Hilfs- und/oder Zusatzstoff, ausgewählt aus der Gruppe enthaltend Öl-in-Wasser-Emulgatoren (O/W-Emulgatoren), Antioxidantien, Gelbildner, Verdickungsmittel, Bindemittel, Filmbildner, Feuchthaltemittel, Duftstoffe.

Geeignete O/W-Emulgatoren sind beispielsweise Natriumstearoylglutamat, Glycerylstearatcitrat, Glycerylstearat, Polyglyceryl-4-Caprate, Glycerylstearat, Ceteareth-20, Ceteareth-12, Cetearylalkohol, Glycol distearate. Bevorzugte Antioxidantien sind Tocopherol und Tocopherolacetat, Betacarotin.

Als Gelbildner können Tonminerale, modifizierte Cellulosen, insbesondere Bentonit, Xanthan, Maltooligosyl-Glucosid zugegeben werden.

Bevorzugte Feuchthaltemittel sind beispielsweise Glycerin, Ethylenglycol, 1,2-Propandiol oder 1,2-Pentandiol.

Weiterhin kann die kosmetische Zubereitung in bevorzugten Ausführungsformen verschiedene Pflanzenextrakte umfassen, beispielsweise Rosa Damascena Flower Water, Cocos Nucifera Fruit Juice, Plantago Ovata Leaf Extract, Aloe Vera Leaf Juice.

Die vorliegende Erfindung bezieht sich insbesondere auch auf die Verwendung von aktiviertem Ultrareinwasser, das einen Gehalt von 60 bis 1500 mg/l ClO⁻ -Ionen und ein Redoxpotential von + 700 bis + 950 mV aufweist, zur Herstellung einer erfindungsgemäßen kosmetischen Zubereitung mit einer wässrigen Phase.

Die erfindungsgemäße kosmetische Zubereitung hat insbesondere den Vorteil, dass sie trotz ihres Gehalts an Wasser ohne oder nur mit einer Konservierung mit Konservierungsstoffen in deutlich reduzierter Konzentration von nur bis zu 35% der zulässigen Höchstkonzentration auskommt.

Weiterhin ist es von Vorteil, dass durch das Weglassen des Konservierungsstoffs oder die Reduktion seiner Konzentration auf bis zu 35% der zulässigen Höchstkonzentration keine Gemische von mehreren unterschiedlichen Konservierungsstoffen in gängigen Konzentrationen eingesetzt werden müssen. Ein durch diese Gemische oft beobachteter sogenannter Cocktaileffekt, bei dem aufgrund der Anzahl verschiedener Konservierungsstoffe trotz jeweils niedriger Konzentration Allergie- und Sensibilisierungs-Probleme bei der Anwendung von kosmetischen Zubereitungen entstehen, kann durch die erfindungsgemäßen Zubereitungen vermieden werden.

Die erfindungsgemäßen kosmetischen Zubereitungen weisen Gemische von Konservierungsstoffen nur dann auf, wenn die unterschiedlichen Konservierungsstoffe durch die Rohstoffe mit in die Zubereitung eingetragen werden. Dieser Eintrag ist nicht zu verhindern, aufgrund der dann vorliegenden Minimalkonzentrationen wird ein nachteiliger Effekt, insbesondere ein Cocktaileffekt, vermieden.

Im Folgenden werden bevorzugte Ausführungsbeispiele der vorliegenden Erfindung beschrieben, ohne dass der Erfindungsgedanke auf diese Beispiele reduziert wird.

### Beispiel 1: Duschgel

| Inhaltsstoff / INCI (EU) | **Menge** [%] |
|---|---|
| AKTIVIERTES ULTRAREINWASSER | 71,80205 |
| SODIUM COCOAMPHOACETATE | 6,30000 |
| COCAMIDOPROPYL BETAINE | 4,26000 |
| DISODIUM LAURETH SULFOSUCCINATE | 4,00000 |
| GLYCERIN | 3,30000 |
| SODIUM CHLORIDE | 2,18000 |
| LAURETH-2 | 1,57500 |
| PEG/PPG-120/10 TRIMETHYLPROPANE TRIOLEATE | 1,57500 |
| PARFUM (FRAGRANCE) | 1,25000 |
| PPG-26-BUTETH-26 | 1,01250 |
| CITRIC ACID | 0,93250 |
| PEG-40 HYDROGENATED CASTOR OIL | 0,65250 |
| COCOS NUCIFERA FRUIT JUICE | 0,49250 |
| PROPYLENE GLYCOL | 0,24625 |
| COCONUT ACID | 0,24000 |
| ETHYLHEXYL METHOXYCINNAMATE | 0,08750 |
| MELIA AZADIRACHTA LEAF EXTRACT | 0,01500 |
| BUTYL METHOXYDIBENZOYLMETHANE | 0,01250 |
| ETHYLHEXYL SALICYLATE | 0,01250 |
| MELIA AZADIRACHTA FLOWER EXTRACT | 0,01250 |
| AMINO-ESTERS-1 | 0,01000 |
| POLYQUATERNIUM-39 | 0,00955 |
| MYRCIARIA DUBIA SEED EXTRACT | 0,00750 |
| COCCINIA INDICA FRUIT EXTRACT | 0,00500 |
| ALOE BARBADENSIS FLOWER EXTRACT | 0,00250 |
| SOLANUM MELONGENA FRUIT EXTRACT | 0,00250 |
| SODIUM BENZOATE * | 0,00190 |
| POTASSIUM SORBATE * | 0,00075 |
| CORALLINA OFFICINALIS EXTRACT | 0,00050 |
| CURCUMA LONGA ROOT EXTRACT | 0,00050 |
| OCIMUM BASILICUM FLOWER/LEAF/STEM EXTRACT | 0,00050 |
| OClMUM SANCTUM LEAF POWDER | 0,00050 |

| | |
|---|---|
| * Konservierungsstoff, der in einer gelieferten Mischung eines Rohstoffs bereits enthalten ist | |

Zur Herstellung eines Duschgels nach Beispiel 1 wird das aktivierte Ultrareinwasser in einem Gefäß bei Raumtemperatur vorgelegt. Es wird kontinuierlich gerührt. Nach und nach werden alle Inhaltsstoffe unter Rühren zugegeben. Das fertige Duschgel wird in ein geeignetes Behältnis abgefüllt.

### Vergleichsbeispiel 1a: Duschgel:

| **Inhaltsstoff / INCI (EU)** | **Menge [%]** |
|---|---|
| WASSER | 71,07205 |
| SODIUM COCOAMPHOACETATE | 6,30000 |
| COCAMIDOPROPYL BETAINE | 4,26000 |
| DISODIUM LAURETH SULFOSUCCINATE | 4,00000 |
| GLYCERIN | 3,30000 |
| SODIUM CHLORIDE | 2,18000 |
| LAURETH-2 | 1,57500 |
| PEG/PPG-120/10 TRIMETHYLPROPANE TRIOLEATE | 1,57500 |
| PARFUM (FRAGRANCE) | 1,25000 |
| PPG-26-BUTETH-26 | 1,01250 |
| CITRIC ACID | 0,93250 |
| PEG-40 HYDROGENATED CASTOR OIL | 0,65250 |
| COCOS NUCIFERA FRUIT JUICE | 0,49250 |
| SODIUM BENZOATE | 0,30190 |
| CHLORPHENESIN | 0,28000 |
| PROPYLENE GLYCOL | 0,24625 |
| COCONUT ACID | 0,24000 |
| POTASSIUM SORBATE | 0,15075 |
| ETHYLHEXYL METHOXYCINNAMATE | 0,08750 |
| MELIA AZADIRACHTA LEAF EXTRACT | 0,01500 |
| BUTYL METHOXYDIBENZOYLMETHANE | 0,01250 |
| ETHYLHEXYL SALICYLATE | 0,01250 |
| MELIA AZADIRACHTA FLOWER EXTRACT | 0,01250 |
| AMINO-ESTERS-1 | 0,01000 |
| POLYQUATERNIUM-39 | 0,00955 |
| MYRCIARIA DUBIA SEED EXTRACT | 0,00750 |
| COCCINIA INDICA FRUIT EXTRACT | 0,00500 |
| ALOE BARBADENSIS FLOWER EXTRACT | 0,00250 |
| SOLANUM MELONGENA FRUIT EXTRACT | 0,00250 |
| CORALLINA OFFICINALIS EXTRACT | 0,00050 |
| CURCUMA LONGA ROOT EXTRACT | 0,00050 |
| OCIMUM BASILICUM FLOWER/LEAF/STEM EXTRACT | 0,00050 |
| OClMUM SANCTUM LEAF POWDER | 0,00050 |

Die Herstellung eines Duschgels nach Vergleichsbeispiel 1a erfolgt analog zu Beispiel 1. Das fertige Duschgel enthält 0,28 % Chlorphenesin. Es wird in ein geeignetes Behältnis abgefüllt.

### Beispiel 2: Lotion

| **Inhaltsstoff / INCI (EU)** | **Menge [%]** |
|---|---|
| Aktiviertes Ultrareinwasser | 45,00 |
| Rosa Damascena Flower Water | 31,5000 |
| Wasser | 16,99 |
| Glycerin | 6,1614 |
| Polyglyceryl-4-caprat | 0,2700 |
| Citronensäure | 0,0750 |
| Sempervivum Tectorum Extrakt | 0,0030 |
| Rhodiola Rosea Root Extrakt | 0,0006 |

Die Herstellung einer Lotion nach Beispiel 2 erfolgt analog zu Beispiel 1.

### Beispiel 3: Gel Creme conventionel

| **Nr.** | **Inhaltsstoff / INCI (EU)** | **Menge [%]** |
|---|---|---|
| 1 | aktiviertes Ultrareinwasser | 59,0 |
| 2 | Aloe Vera-Blätterextrakt | 20,0 |
| 3 | Gelbildner (Bentonit, Xanthan) | 1,8 |
| 4 | Stabilisator (Citronensäure) | 0,1 |
| 5 | O/W-Emulgator (Natriumstearolyglutamat) | 0,1 |
| 6 | Konservierungsstoff Chlorphenesin | 0,1 |
| 7 | Dicaprylylether | 6,0 |
| 8 | O/W-Emulgator (Glyceryl Stearat Citrat) | 4,5 |
| 9 | Antioxidant Tocopherol und Salze | 0,02 |
| 10 | Feuchthaltemittel (Natriumhyaluronat) | 0,1 |
| 11 | aktiviertes Ultrareinwasser | 5,08 |
| 12 | Bindemittel (Maltooligosylglucosid, Stärkehydrolysat) | 2,0 |
| 13 | Parfüm, Duftstoffe | 0,2 |
| 14 | 1,2-Pentandiol | 1,0 |

Zur Herstellung einer Hautcreme nach Beispiel 3 werden die Inhaltsstoffe Nr. 1 bis 6 der wässrigen Phase unter langsamem Rühren auf Stufe 1 einer Emulgiermaschine Turbine V2 auf 75 °C erwärmt. Getrennt hiervon werden die Inhaltsstoffe Nr. 7 bis 9 der öligen Phase ebenfalls unter langsamem Rühren auf 75 °C erwärmt. Die beiden Phasen werden zusammengegeben und unter schnellem Rühren auf Stufe 2 mit ungefähr 3000 rpm in der Emulgiermaschine Turbine V2 emulgiert und nach 5 min wird die gebildete Emulsion auf Raumtemperatur abgekühlt.

Der Inhaltsstoff Nr. 10 wird in Inhaltsstoff Nr. 11 für 30 min quellen gelassen. Im Anschluss wird die gequollene Mischung unter Rühren zur Emulsion gegeben. Sobald die Mischung gut eingearbeitet ist und die Emulsion ein homogenes Aussehen hat, wird der Inhaltsstoff Nr. 12 nach und nach zugegeben, wobei ohne Unterbrechung gerührt wird. Nach kurzem Aufrühren von Inhaltsstoff Nr. 13 wird dieser ebenfalls zur Emulsion gegeben. Schließlich wird Inhaltsstoff Nr. 14 zugegeben. Wenn notwendig, wird der pH-Wert mit einem pH-Regulator, z.B. Citronensäure, auf einen Wert zwischen 5,0 bis 5,5 eingestellt. Die fertige Emulsion wird in ein geeignetes Behältnis abgefüllt.

Als Vergleichsbeispiel 3a wird eine Emulsion wie unter Beispiel 3 beschrieben hergestellt, mit dem einzigen Unterschied, dass statt des aktivierten Ultrareinwassers (Inhaltsstoff Nr. 1) ein für kosmetische Zubereitungen übliches gereinigtes Wasser (Aqua purificata) entsprechend dem Inhaltsstoff Nr. 10 in der entsprechenden Menge zugegeben wird. Die fertige Emulsion wird in ein geeignetes Behältnis abgefüllt.

Weiterhin wird als zusätzliches Vergleichsbeispiel 3b eine Emulsion wie unter Beispiel 3 beschrieben hergestellt, mit dem Unterschied, dass statt des aktivierten Ultrareinwassers (Inhaltsstoff Nr. 1) ein für kosmetische Zubereitungen übliches gereinigtes Wasser (Aqua purificata) entsprechend dem Inhaltsstoff Nr. 10 in der entsprechenden Menge zugegeben wird. Weiterhin wird der Konservierungsstoff Nr. 6, Chlorphenesin, in einer Konzentration von 0,28 % zugegeben. Diese Konzentration liegt nahe der zulässigen Höchstkonzentration für Chlorphenesin von 0,1 %. Die Menge des Wassers Nr. 1 wird entsprechend angepasst. Die fertige Emulsion wird in ein geeignetes Behältnis abgefüllt.

### Beispiel 4: Gel Creme organic

| **Nr.** | **Inhaltsstoff / INCI (EU)** | **Menge [%]** |
|---|---|---|
| 1 | aktiviertes Ultrareinwasser | 59,0 |
| 2 | Aloe Vera-Blätterextrakt | 20,0 |
| 3 | Gelbildner (Bentonit, Xanthan) | 1,8 |
| 4 | O/W-Emulgator (Natriumstearolyglutamat) | 0,1 |
| 5 | Dicaprylylether | 6,0 |
| 6 | O/W-Emulgator (Glyceryl Stearat Citrat) | 4,5 |
| 7 | Antioxidant (Tocopherol und Salze) | 0,02 |
| 8 | Feuchthaltemittel (Natriumhyaluronat) | 0,1 |
| 9 | Gereinigtes Wasser | 5,15 |
| 10 | Maltooligosylglucosid, Stärkehydrolysat | 2,0 |
| 11 | Konservierungsstoff (Na-benzoat + K-sorbat) | 0,13 |
| 12 | Parfüm, Duftstoffe | 0,2 |
| 13 | 1,2-Propandiol | 1,0 |

Analog Beispiel 3 werden für eine Hautcreme nach Beispiel 4 die die Inhaltsstoffe Nr. 1 bis 4 und Nr. 11 der wässrigen Phase unter langsamem Rühren auf Stufe 1 auf 75 °C erwärmt. Getrennt hiervon werden die Inhaltsstoffe Nr. 5 bis 7 der öligen Phase ebenfalls unter langsamem Rühren auf 75 °C erwärmt. Die beiden Phasen werden zusammengegeben und unter schnellem Rühren mit ungefähr 3000 rpm in einer Emulgiermaschine Turbine V2 emulgiert und nach 5 min wird die gebildete Emulsion auf Raumtemperatur abgekühlt.

Der Inhaltsstoff Nr. 8 wird in Inhaltsstoff Nr. 9 für 30 min quellen gelassen. Im Anschluss wird die gequollene Mischung unter Rühren zur Emulsion gegeben. Sobald die Mischung gut eingearbeitet ist und die Emulsion ein homogenes Aussehen hat, wird der Inhaltsstoff Nr. 10 nach und nach zugegeben, wobei ohne Unterbrechung gerührt wird. Nach kurzem Aufrühren von Inhaltsstoff Nr. 12 wird dieser ebenfalls zur Emulsion gegeben. Schließlich wird Inhaltsstoff Nr. 13 zugegeben. Wenn notwendig, wird der pH-Wert mit einem pH-Regulator, z.B. Citronensäure, auf einen Wert zwischen 5,0 bis 5,5 eingestellt. Die fertige Emulsion wird in ein geeignetes Behältnis abgefüllt.

Als Vergleichsbeispiel 4a wird eine Emulsion wie unter Beispiel 4 beschrieben hergestellt, mit dem einzigen Unterschied, dass statt des aktivierten Ultrareinwassers (Inhaltsstoff Nr. 1) ein für kosmetische Zubereitungen übliches gereinigtes Wasser (Aqua purificata) entsprechend dem Inhaltsstoff Nr. 9 in der entsprechenden Menge zugegeben wird. Die fertige Emulsion wird in geeignete Behältnisse abgefüllt.

Weiterhin wird als zusätzliches Vergleichsbeispiel 4b eine Emulsion wie unter Beispiel 4 beschrieben hergestellt, mit dem Unterschied, dass statt des aktivierten Ultrareinwassers (Inhaltsstoff Nr. 1) ein für kosmetische Zubereitungen übliches gereinigtes Wasser (Aqua purificata) entsprechend dem Inhaltsstoff Nr. 9 in der entsprechenden Menge zugegeben wird. Weiterhin werden die Konservierungsstoffe Nr. 11, in einer Konzentration von 1,0 % zugegeben. Diese Konzentration liegt nahe der zulässigen Höchstkonzentration für Natriumbenzoat von 0,5 % und Kaliumsorbat von 0,6 %. Die Menge des Wassers Nr. 1 wird entsprechend angepasst. Die fertige Emulsion wird in ein geeignetes Behältnis abgefüllt.

### Mikrobiologischer Test:

Um die mikrobiologische Stabilität der kosmetischen Zubereitungen nach den Beispielen 1 bis 4 und den Vergleichsbeispielen 3a, 3b, 4a und 4b zu testen, werden jeweils Proben genommen.

Die Evaluierung der Proben zum Nachweis des antimikrobiellen Schutzes in den kosmetischen Produkten wird bei einem Labor in Auftrag gegeben. Es werden sogenannte Konservierungsbelastungstests (Challenge-Tests) gemäß des Standards NF EN ISO 11930:Februar 2019 durchgeführt.

Zu Testbeginn erfolgt eine absichtliche Inokulation der Proben der kosmetischen Zubereitung mit fünf verschiedenen Stämmen von Mikroorganismen (Staphylococcus aureus, Pseudomonas aeruginosa, Escherichia coli, Candida albicans und Aspergillus Brasiliensis). Die Ausgangskeimzahlen werden ermittelt und die beimpften Proben unter definierten Bedingungen eingelagert. Nach festgelegten Zeitpunkten (7 Tage, 14 Tage und 28 Tage) werden die Keimzahlen der beimpften Proben bestimmt, um die Entwicklung der Keimzahlen über den jeweiligen Testzeitraum zu verfolgen. Dabei wird überprüft, ob die Keimzahlen entsprechend reduziert werden. Da nach dem Öffnen einer kosmetischen Zubereitung im Gebrauch mit einem Eintrag von Mikroorganismen gerechnet werden muss, gibt das Ergebnis dieses Tests einen Hinweis hinsichtlich der mikrobiellen Stabilität der kosmetischen Zubereitung.

Um die Kriterien nach ISO 11930 zu erfüllen, müssen nach 7, 14 und 28 Tagen folgende logarithmische Reduktionswerte erreicht werden; hierbei werden für Aspergillus brasiliensis nach 7 Tagen keine Werte bestimmt, sondern erst nach 14 und nach 28 Tagen:

**Tab. 2: geforderte logarithmische Reduktionswerte nach ISO 11930**

| **Geforderte logarithmische Reduktionswerte (a)** | | | | | |
|---|---|---|---|---|---|
| **Kriterium A** | **0 h** | **2d** | **7d** | **14 d** | **28 d** |
| Bakterien | --- | --- | ≥ 3 | ≥ 3 (b) | ≥ 3 (b) |
| C. albicans | --- | --- | ≥ 1 | ≥ 1 (b) | ≥ 1 (b) |
| Aspergillus brasiliensis | --- | --- | --- | Kein Anstieg gegenüber dem Oh-Wert | ≥ 1 (b) |

| **Kriterium B** | **0 h** | **2d** | **7d** | **14 d** | **28 d** |
|---|---|---|---|---|---|
| Bakterien | --- | --- | --- | ≥ 3 | ≥ 3 (b) |
| C. albicans | --- | --- | --- | ≥ 1 | ≥ 1 (b) |
| Aspergillus brasiliensis | --- | --- | --- | Kein Anstieg gegenüber dem Oh-Wert | Kein Anstieg gegenüber dem 14d-Wert |

| | | | | | |
|---|---|---|---|---|---|
| (a) eine Abweichung von 0,5 log-Stufen ist zulässig (b) kein Anstieg gegenüber der letzten Zählung | | | | | |

Wenn nach der ISO 11930 das Kriterium A erfüllt wird, kann man laut der Norm davon ausgehen, dass das Produkt vor mikrobieller Vermehrung geschützt ist. Sollte ausschließlich Kriterium B erreicht werden, müssen weitere Sicherheitsmaßnahmen wie z.B. eine Schutzverpackung ergriffen werden, um das mikrobielle Risiko während des Gebrauchs entsprechend zu verringern und die Anforderungen der Norm zu erfüllen.

Die Ergebnisse sind in folgender Tabelle 3 zusammengefasst.

**Tab. 3: Ergebnisse der Konservierungsbelastungstest Beispiele, Vergleichsbeispiele**

| | Keim (alle bezogen von ATTC) | | | | |
|---|---|---|---|---|---|
| | Staphylococcus aureus | Pseudomonas aeruginosa | Escherichia coli | Candida albicans | Aspergillus Brasiliensis |
| Logarithmische Reduktion nach t=7 | | | | | |
| Beispiel 1 | ≥ 3 | ≥ 3 | ≥ 3 | ≥ 1 | |
| Vergleichsbeispiel 1a | ≥ 3 | ≥ 3 | ≥ 3 | ≥ 1 | |
| Beispiel 2 | > 3 | > 3 | > 3 | ≥ 1 | |
| Beispiel 3 | > 4,5 | > 4,8 | > 4,8 | > 3,7 | |
| Vergleichsbeispiel 3a | 0,4 | 0,4 | 3,2 | 0,3 | |
| Vergleichsbeispiel 3b | > 4,5 | > 4,7 | > 4,7 | > 3,8 | |
| Beispiel 4 | > 4,7 | > 4,7 | > 4,8 | > 3,7 | |
| Vergleichsbeispiel 4a | Entsprach nicht den Kriterien A oder B nach 7 Tagen | | | | |
| Vergleichsbeispiel 4b | > 4,5 | > 4,8 | > 4,8 | 1,5 | |

| Logarithmische Reduktion nach t=14 | | | | | |
|---|---|---|---|---|---|
| Beispiel 1 | ≥ 3 na | ≥ 3 na | ≥ 3 na | ≥ 1 na | ≥ 0 |
| Vergleichsbeispiel 1a | ≥ 3 na | ≥ 3 na | ≥ 3 na | ≥ 1 na | ≥ 0 |
| Beispiel 2 | > 3 | > 3 | > 3 | > 1 | 0,2 |
| Beispiel 3 | > 4,5 | > 4,8 | > 4,8 | > 3,7 | > 3,4 |
| Vergleichsbeispiel 3a | 0,4 | 0,4 | 4,9 | -0,5 | 0,4 |
| Vergleichsbeispiel 3b | > 4,5 | > 4,7 | > 4,7 | > 3,8 | 3,0 |
| Beispiel 4 | > 4,7 | > 4,7 | > 4,8 | > 3,7 | > 3,4 |
| Vergleichsbeispiel 4a | Entsprach nicht den Kriterien A oder B nach 7 Tagen (T=7), daher nach 14 Tagen nicht getestet | | | | |
| Vergleichsbeispiel 4b | > 4,5 | > 4,8 | > 4,8 | > 3,7 | 0,1 |

| | Keim (alle bezogen von ATTC) | | | | |
|---|---|---|---|---|---|
| | Staphylococcus aureus | Pseudomonas aeruginosa | Escherichia coli | Candida albicans | Aspergillus Brasiliensis |
| Logarithmische Reduktion nach t=28 | | | | | |
| Beispiel 1 | ≥ 3 na | ≥ 3 na | ≥ 3 na | ≥ 1 na | ≥ 1 na |
| Vergleichsbeispiel 1a | ≥ 3 na | ≥ 3 na | ≥ 3 na | ≥ 1 na | ≥ 1 na |
| Beispiel 2 | > 3 | > 3 | > 3 | > 1 | 0,5 |
| Beispiel 3 | > 4,5 | > 4,8 | > 4,8 | > 3,7 | > 3,4 |
| Vergleichsbeispiel 3a | Entsprach nicht den Kriterien A oder B nach 14 Tagen (T=14), daher nach 28 Tagen nicht getestet | | | | |
| Vergleichsbeispiel 3b | > 4,5 | > 4,7 | > 4,7 | > 3,8 | > 3,5 |
| Beispiel 4 | > 4,7 | > 4,7 | > 4,8 | > 3,7 | > 3,4 |
| Vergleichsbeispiel 4a | Entsprach nicht den Kriterien A oder B nach 7 Tagen (T=7), daher nach 28 Tagen nicht getestet | | | | |
| Vergleichsbeispiel 4b | > 4,5 | > 4,8 | > 4,8 | > 3,7 | 0,9 |

Die Beispiele 3, 4 und die Vergleichsbeispiele 3a, 3b, 4a und 4b werden ebenso einem Challenge Test unterzogen, bei dem eine Lagerung über 28 Tage bei Raumtemperatur und außerdem über 28 Tage bei 50 °C erfolgt. Es zeigen:
- Figur 1: ein Netzdiagramm mit Ergebnissen des Challengetest (T=28) nach 28 Tagen bei Raumtemperatur für Beispiel 3, Vergleichsbeispiele 3a und 3b
- Figur 2: ein Netzdiagramm der Ergebnisse eines Challengetest (T=28) nach 28 Tagen bei T = 50 °C für Beispiel 3, Vergleichsbeispiele 3a und 3b
- Figur 3: ein Netzdiagramm der Ergebnisse eines Challengetest (T=28) nach 28 Tagen bei Raumtemperatur für Beispiel 4, Vergleichsbeispiele 4a und 4b
- Figur 4: ein Netzdiagramm der Ergebnisse eines Challengetest (T=28) nach 28 Tagen bei 50 °C für Beispiel 4, Vergleichsbeispiele 4a und 4b

Es zeigt sich, dass die erfindungsgemäßen Zubereitungen nach Beispiel 3 und 4 sowie die Zubereitungen nach Vergleichsbeispiel 3b und 4b mit Konservierungsstoff in der zulässigen Höchstkonzentration die nach ISO 11930 vorgegebenen Reduktionswerte problemlos einhalten. Die Vergleichsbeispiele 3a und 4a, bei denen Konservierungsstoff in 30 bzw. 33 % der zulässigen Höchstkonzentration zugegeben wurde, aber nicht das aktivierte Ultrareinwasser, erfüllen die Kriterien A und B nach ISO 11930 nicht.

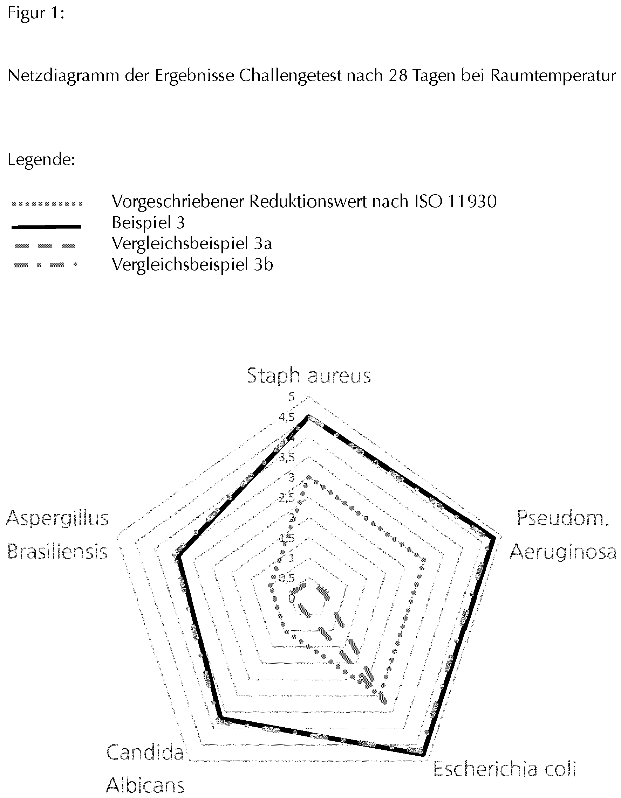

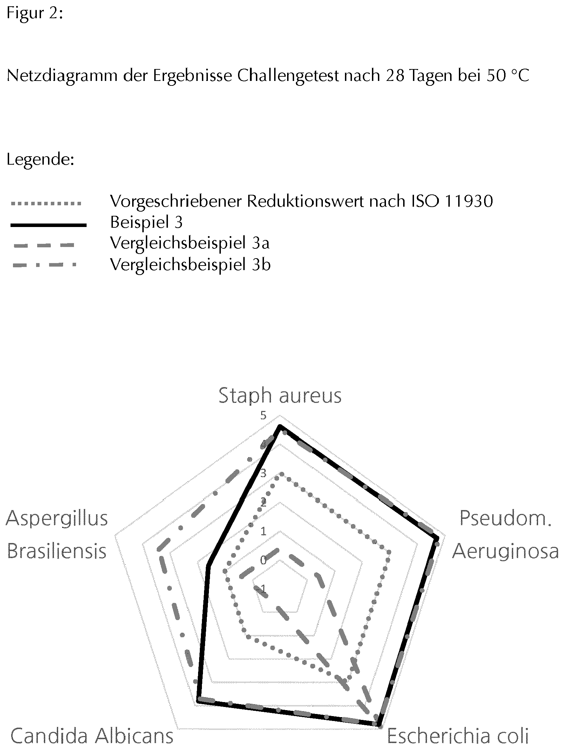

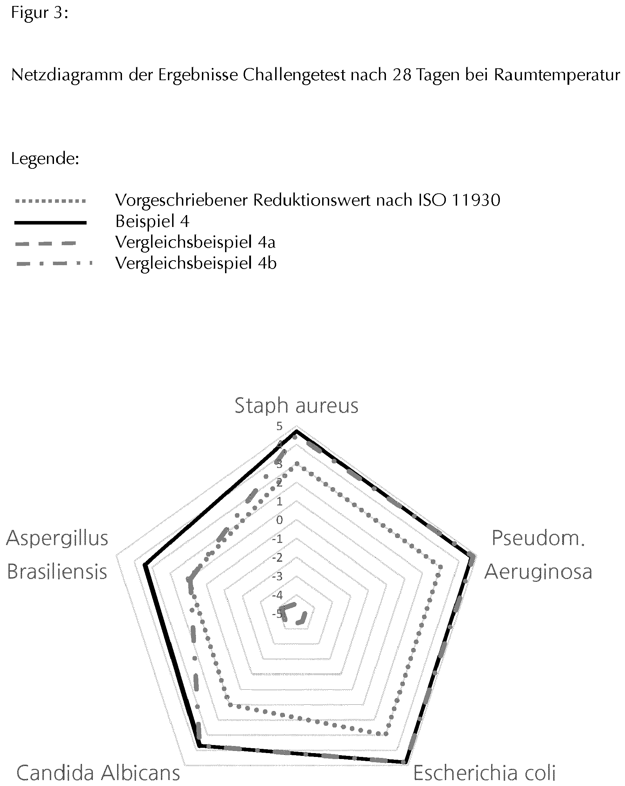

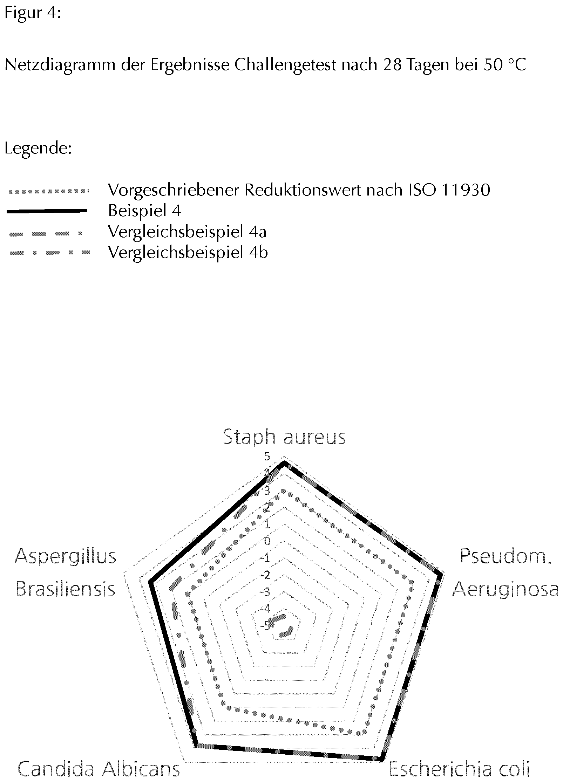

Wie den Ergebnissen, insbesondere der Tabelle 3 und den Figuren 1 bis 4, zu entnehmen ist, weisen die erfindungsgemäßen kosmetischen Zubereitungen die geforderten Reduktionswerte nach dem Kriterium A auf. Sie sind somit trotz der nur in geringen Mengen enthaltenen Konservierungsstoffe ausreichend gegen mikrobiellen Verderb geschützt und sind in der Anwendung für den Verbraucher sicher.

Es müssen keine zusätzlichen Maßnahmen, wie eine Schutzverpackung oder ähnliches, ergriffen werden, um die Zubereitung von mikrobiellem Risiko zu bewahren. Dies macht eine anwenderfreundlichere und kostengünstigere Formulierung der kosmetischen Zubereitung möglich

## Patentansprüche

1. Kosmetische Zubereitung enthaltend eine wässrige Phase,
wobei die wässrige Phase Wasser und ein aktiviertes Ultrareinwasser umfasst, wobei der wässrigen Phase das Wasser und das aktivierte Ultrareinwasser zugegeben werden, und wobei das aktivierte Ultrareinwasser vor der Zugabe zur wässrigen Phase einen Gehalt von 60 bis 1500 mg/l ClO⁻ -Ionen und ein Redoxpotential von + 700 bis + 950 mV aufweist,
wobei der Anteil an aktiviertem Ultrareinwasser mindestens 70 bis 99,9% des zu der wässrigen Phase insgesamt zugegebenen Wassers und aktivierten Ultrareinwassers beträgt.

2. Kosmetische Zubereitung nach Anspruch 1,
wobei die wässrige Phase einen Konservierungsstoff umfasst,
wobei die Konzentration des Konservierungsstoffs in der kosmetischen Zubereitung bis zu 35 % einer zulässigen Höchstkonzentration in Gew.-% beträgt.

3. Kosmetische Zubereitung nach Anspruch 2,
wobei die Konzentration des Konservierungsstoffs in der kosmetischen Zubereitung bis zu 18 % der zulässigen Höchstkonzentration beträgt.

4. Kosmetische Zubereitung nach Anspruch 2,
wobei die Konzentration des Konservierungsstoffs in der kosmetischen Zubereitung 7,5 bis 34 % der zulässigen Höchstkonzentration beträgt.

5. Kosmetische Zubereitung nach einem der vorhergehenden Ansprüche 2 bis 4,
wobei die Konzentration des Konservierungsstoffs in der kosmetischen Zubereitung 7,5 bis 18 % der zulässigen Höchstkonzentration beträgt.

6. Kosmetische Zubereitung nach einem der vorhergehenden Ansprüche, wobei das aktivierte Ultrareinwasser einen pH-Wert von 8,2 - 9,5 aufweist.

7. Kosmetische Zubereitung nach einem der vorhergehenden Ansprüche, wobei die Zubereitung eine ölige Phase umfasst und wobei die ölige Phase mit der wässrigen Phase eine Öl-in-Wasser-Emulsion bildet.

8. Kosmetische Zubereitung nach einem der vorhergehenden Ansprüche, wobei die wässrige Phase ein Lösungsmittel ausgewählt aus der Gruppe enthaltend Ethanol, Isopropanol, Glycerol, Propylenglycol, Butylenglycol Pentylenglycol und Hexylenglycol umfasst.

9. Kosmetische Zubereitung nach einem der vorhergehenden Ansprüche, wobei die Zubereitung einen weiteren Hilfs- und/oder Zusatzstoff umfasst, ausgewählt aus der Gruppe enthaltend Öl-in-Wasser-Emulgatoren (O/W-Emulgatoren), Antioxidantien, Gelbildner, Verdickungsmittel, Bindemittel, Filmbildner, Feuchthaltemittel und Duftstoffe.

10. Verwendung von aktiviertem Ultrareinwasser, das einen Gehalt von 60 bis 1500 mg/l ClO⁻ -Ionen und ein Redoxpotential von + 700 bis + 950 mV aufweist, zur Herstellung einer kosmetischen Zubereitung mit einer wässrigen Phase gemäß einem der Ansprüche 1 bis 9.
